Europäisches Patentamt

European Patent Office

Office européen des brevets

(11) Publication number: **0 301 835**
**A1**

(12) **EUROPEAN PATENT APPLICATION**

(21) Application number: 88306926.2

(22) Date of filing: 27.07.88

(51) Int. Cl.⁴: **C 12 P 21/02**

(30) Priority: 29.07.87 US 79666

(43) Date of publication of application:
01.02.89 Bulletin 89/05

(84) Designated Contracting States: **ES GR**

(71) Applicant: **Schering Biotech Corporation**
**901 California Avenue**
**Palo Alto California 94304-1104 (US)**

(72) Inventor: **Kastelein, Robert A.**
**463 Summit Drive**
**Redwood City California 94062 (US)**

**van Kimmenade, Anita M.A.**
**201 Loma Verde Street No. H**
**Palo Alto California 94306 (US)**

(74) Representative: **Ritter, Stephen David et al**
**Mathys & Squire 10 Fleet Street**
**London EC4Y 1AY (GB)**

(54) Purification of human interleukin-4 expressed in Escherichia Coli.

(57) A method is provided for refolding and purifying biologi-
cally active human interleukin-4 from insoluble aggregates in
bacteria. The method comprises solubilizing the aggregates
with an aqueous solution containing a chaotropic agent and a
reducing agent, followed by steps of controlled and sequential
reduction in the concentrations of the two agents to form
biologically active human interleukin-4. Finally the biologically
active human interleukin-4 is separated from inactive material
by standard biochemical procedures.

**Description**

## PURIFICATION OF HUMAN INTERLEUKIN-4 EXPRESSED IN ESCHERICHIA COLI

### Field of Invention

The invention relates generally to methods for purifying proteins, and more particularly, to methods for purifying human interleukin-4 expressed in aggregated form in Escherichia coli.

### BACKGROUND

Human interleukin-4 (IL-4) was first cloned and characterized by Yokota et al., Proc. Natl. Acad. Sci., Vol. 83, pgs. 5894-5898 (1986). IL-4 is a highly pleiotropic lymphokine which affects many different components of the immune system. It has T cell growth factor (TCGF) activity; and B cell growth factor activity (BCGF). It is capable of potentiating the TCGF activity of interleukin-2 (IL-2) and the colony forming activity of granulocyte-macrophage colony stimulating factor. It induces the preferential production of IgG₁ and IgE, and induces the expression of human leukocyte class II DR antigens. These activities suggest several possible therapeutic uses, e.g. as a potentiating agent for IL-2 anticancer therapy, as a potentiating agent for GM-CSF stimulated bone marrow regeneration, or as an agent to treat bare lymphocyte syndrome, Touraine, Lancet, pgs. 319-321 (February 7, 1981); and Sullivan et al., J. Clin. Invest., Vol. 76, pgs. 75-79 (1985).

In the production of genetically engineered proteins such as IL-4, separation of the expressed protein from the transformed host cells or their culture supernatants can be a major problem, Dwyer, Biotechnology, Vol. 2, pg. 957 (1984). Frequently, mammalian proteins overexpressed in bacterial cells form precipitates (which are also sometimes referred to as "aggregates," or "refractile bodies," or "inclusion bodies"), e.g. Koths et al., U.S. Patent 4,569,790; Langley et al., Eur. J. Biochem., Vol. 163, pgs. 313-321 (1987); Winkler et al., Biotechnology, Vol. 3, pgs. 990-998 (1985); and De Lamarter et al., The EMBO Journal, Vol. 4, pgs. 2575-2581 (1985). Such aggregates are associated with the improper formation of intermolecular bonds, e.g. hydrogen bonds and/or disulfide bonds between polypeptides. The general approach to obtaining biologically active polypeptides from such aggregates involves first treating with chaotropic agents and reducing agents to solubilize the aggregates, followed by a controlled removal of the agents to permit the polypeptides to refold into their biologically active conformation while minimizing the reformation of aggregates. The success of such a procedure depends critically on the nature of the expressed polypeptide, the selection of the chaotropic agents and reducing agents, and the conditions under which the solubilized and denatured polypeptides are allowed to refold into their biologically active states, e.g. White, Methods in Enzymology, Vol. 11, pgs. 481-484 (1967); Wetlaufer, Methods in Enzymology, Vol. 107, pgs. 301-304 (1984); and Means and Feeney, Chemical Modification of Proteins (Holden-Day, San Francisco, 1971).

In view of the fact that human IL-4 is expressed as insoluble aggregates in E. coli, it would be highly desirable if an efficient method were available for extracting and purifying biologically active human IL-4 from such aggregates.

### SUMMARY OF THE INVENTION

The invention is a method for recovering biologically active human IL-4 from bacteria that produce human IL-4 as insoluble intracellular aggregates. The method comprises the steps of (i) disrupting the cell membrane of the bacteria; (ii) separating the aggregates from the disruptate; (iii) solubilizing the aggregates with an aqueous solution containing a chaotropic agent and a reducing agent to form reduced and denatured IL-4; (iv) diluting the aqueous solution containing the reduced and denatured IL-4 so that the concentration of the chaotropic agent is reduced and so that the average intermolecular distance between reduced and denatured IL-4 molecules is increased between about 1.5 to about 2.5 fold; (v) reducing the concentration of the chaotropic agent and the concentration of the reducing agent so that the reduced and denatured IL-4 renatures into a biologically active conformation; and (vi) purifying the biologically active IL-4 of step (v). Preferably, prior to purification, the renatured IL-4 is concentrated, and the preferred chaotropic agent is guanidine.

### Brief Description of the Drawings

Figure 1 is a diagrammatic representation of E. coli expression vector TRPC11 with a human IL-4 cDNA insert.

Figure 2 graphically illustrates the gel filtration elution profile of refolded human IL-4.

Figure 3 illustrates western blots of IL-4 proteins after various methods of denaturation and separation by gel electrophoresis.

### DETAILED DESCRIPTION OF THE INVENTION

The invention is a method for purifying human IL-4 produced in aggregate form by a genetically transformed bacteria particularly E. coli. As used herein, the term "transformed bacteria" means a bacteria that has been genetically engineered to produce human IL-4. Such genetic engineering usually entails the introduction of an expression vector into the bacteria which is capable of autonomous replication and protein expression with respect to genes in the bacterial genome. Construction of bacterial expression vectors is well known in the art, once the nucleotide sequence encoding a desired protein is known or otherwise available, e.g. DeBoer in U.S. Patent 4,551,433 discloses promoters for use in bacterial expression vectors; Goeddel et al., in U.S. Patent 4,601,980, and Riggs, in U.S. Patent 4,431,739 discloses the production of mammalian proteins by E. coli expression systems; and Riggs (cited above), Ferretti et al., Proc. Natl. Acad. Sci., Vol. 83, pgs. 599-603 (1986), Sproat et al., Nucleic Acids Research, Vol. 13, pgs. 2959-2977 (1985), and Mullenbach et al., J. Biol. Chem., Vol. 261, pgs. 719-722 (1986) disclose how to construct synthetic genes for expression in bacteria. Accordingly, these references are incorporated by reference. The amino acid sequence of mature human IL-4 is disclosed by Yokota et al. (cited above), and the cDNA encoding human IL-4 carried by the pcD vector described in Yokota et al. (cited above) is deposited with the American Type Culture Collection (ATCC), Rockville, MD, under accession number 53337. Many bacterial expression vectors and hosts are available commercially and through the ATCC. E. coli is the preferred bacterial host.

The transformed bacteria are grown in a suitable growth medium, typically to an optical density (OD) of at least about 30 at 680 nm, and preferably between about 20 and 40 at 680 nm. The composition of the growth medium will depend upon the particular bacteria involved. The medium is an aqueous medium containing compounds that fulfill the nutritional requirements of the bacteria. Growth media will typically contain assimilable sources of carbon and nitrogen, energy sources, magnesium, potassium and sodium ions, and optionally amino acids and purine and pyrimidine bases. (See Review of Medical Biology, Lange Medical Publications, 14th Ed pgs. 80-85 (1980)). Growth media for E. coli are well known in the art.

After the cells are harvested from the culture, they may be concentrated, if necessary, to about 20 to 150 mg/ml, preferably 80 to 100 mg/ml (OD 40 to 300, preferably 160 to 200 at 680 nm) by filtration, centrifugation, or other conventional methods.

Following concentration, the cell membranes of the bacteria are disrupted. The main purpose of the disruption is to facilitate the following separation and solubilization steps. Conventional cell disruption techniques such as homogenization, sonication, or pressure cycling may be used in this step of the process. Preferred methods are sonication or homogenization with a Manton-Gaulin homogenizer. The end point of the disruption step may be monitored by optical density, with the optical density of the suspension typically decreasing about 65% to 85%. In any event, the disruption should break substantially all of the cells so that substantially no intact cells are carried through to the solubilization step. Before the disruption, the pH of the liquid phase of the concentration is adjusted, if necessary, to a level that facilitates removal of E. coli proteins (i.e. contaminant proteins) in subsequent steps, while retaining IL-4 protein as an insoluble complex in the cellular debris. The pH may be so adjusted by adding suitable buffers. In most instances pHs in the range of about 7 to about 9 will be used.

After disruption and up to the dialysis step it is preferable that the solutions containing the human IL-4 also contain protease inhibitors, such as ethylenediaminetetraacetic acid (EDTA), phenylmethylsulfonyl fluoride (PMSF), or the like, to prevent endogenous bacterial proteases from degrading the human IL-4. Dialysis, concentration, and purification are preferably carried out at 4°C primarily to reduce the possibility of degradation by proteases.

After the cells have been disrupted the particulate matter is separated from the liquid phase of the disruptate and resuspended in an aqueous medium. The particulate matter may optionally be washed with buffer at this stage to remove any water soluble E. coli proteins therein. In any event, the protein concentration of the cell suspension subjected to the extraction will usually be in the range of about 5 to about 60 mg/ml, preferably 20 to 40 mg/ml.

After separating the aggregates of IL-4 from the disruptate, they are treated with an aqueous solution (referred to as the "first aqueous solution") containing a chaotropic agent and a reducing agent in order to solubilize the aggregates. As used herein, "chaotropic agent" refers to a protein denaturant that dissociates hydrogen bonds (thereby disrupting the native secondary and tertiary structures) while at the same time leaves primary structure unaffected. Preferred chaotropic agents include urea and guanidine hydrochloride. The most preferred chaotropic agent is guanidine hydrochloride at a concentration between 3-6 moles/liter (M). As used herein "reducing agent" refers to compounds capable of reducing disulfide bonds. Preferably, the reducing agents have low molecular weights so that they can be separated from IL-4 using standard dialysis procedures. Reducing agents include glutathione (especially combinations of the reduced and oxidized forms), dithiothreitol, betamercaptoethanol, thioglycolate, sodium borohydrate, N-acetyl cysteine, cysteine, and the like. Preferred reducing agents are thiol reducing agents, such as glutathione and dithiothreitol. The most preferred reducing agent is glutathione.

When guanidine and glutathione are used as the chaotropic and reducing agents, respectively, it is preferable that the initial concentration of aggregates in the first solution be less than or equal to about 2.5 milligrams/milliliter (mg/ml). Otherwise, precipitates form when the reducing and chaotropic agents are used at their preferred concentrations.

Since the reactive component of a thiol reducing agent is the thiolate ion, it is preferable that the reduction/denaturation step take place at alkaline pH. More preferably, the reducing agent is used between pH 7.0 to pH 9.0. Most preferably, when the reducing agent is glutathione, the pH is maintained at about 8. And preferably, the reduced form of glutathione is present at a concentration of between about 1-3 millimoles/liter (mM), and the oxidized form of glutathione is present at about one-fifth to one-tenth that concentration. Preferably the reduction takes place within the temperature range of 25-40°C for between about 1-4 hours.

After the IL-4 in the aggregates is denatured and reduced, the solution is diluted to sufficiently lower the protein concentration so that during the refolding process the formation of intramolecular hydrogen and disulfide bonds is favored over the formation of intermolecular bonds. The dilution should not be so great that appreciable amounts of protein are lost by non-specific binding to container walls, or the like. Preferably, the aqueous solution is diluted by a factor of about 5-10 with a second aqueous solution which contains a reducing agent but not a chaotropic agent, e.g. the second aqueous solution may be identical to the first aqueous solution except that no chaotropic agent is present. Also, it is preferable that the second aqueous solution contain salts at approximately a physiological concentration (e.g. between about 50-200 mM, or greater). This will prevent re-aggregation through ionic interactions between the proteins.

The particular extent of dilution can vary a great deal. The objective of the dilution step is to ensure that the average time between intramolecular interactions, e.g. collisions between different parts of the same polypeptide, is much less than the average time between intermolecular interactions, e.g. collisions between different polypeptides. Average intermolecular distance (at a particular instant of time) is a measure of the average time between intermolecular collisions, provided temperature remains constant. Moreover, changes in average intermolecular distance are monotonically related to changes in dilution. Thus, the dilution step of the invention may be characterized by its effect on average intermolecular distances, or vice versa. Changes in concentration resulting from dilution are readily converted into changes in average intermolecular distance by assuming a Poisson distribution of molecules. Thus, a 2 fold dilution increases average intermolecular distance by a factor of about 1.27 and a 10 fold dilution increases average intermolecular distance by a factor of about 2.17 fold. Preferably, at room temperature the dilution step results in an increase by a factor of 1.5-2.5 in the average intermolecular distance between human IL-4 molecules.

After dilution the resulting solution is dialyzed to reduce the concentrations of the chaotropic agent and reducing agent by a factor of about a thousand. For example, a liter solution of the reduced and denatured IL-4 can be dialysed 3 times against 10 liters of an aqueous solution buffered at pH between 7 and 9. The dialysis removes the reducing agent and the chaotropic agent, and permits the formation of intramolecular disulfide bonds and correctly folded human IL-4.

Finally, the soluble and biological active IL-4 is separated from contaminants in the dialyzate (the material retained by the dialysis bag) by standard chromatographic separation procedures. This purification step may be preceeded by a concentration step using standard ultrafiltration membranes, e.g. Amicon YM5. Preferably, gel filtration chromatography, high performance liquid chromatography (HPLC), or a combination of gel filtration chromatography and HPLC are used to purify the biologically active human IL-4. Most preferably the dialyzate after concentration is subjected to gel filtration chromatography using a phosphate buffer, at pH between about 6.5 and about 8.5, containing a physiological concentration of salts, such as 50-200 mM NaCl.

Biological activity of IL-4 is measured by its ability to stimulate and maintain the growth of T cells, i.e. by T cell growth factor (TCGF) activity. Several standard assays have been described for TCGF activity, e.g. Devos et al., Nucleic Acids Research, Vol. 11, pgs. 4307-4323 (1983); Thurman et al., J. Biol. Response Modifiers, Vol. 5, pgs 85-107 (1986); and Robert-Guroff et al., Chapter 9 in Guroff, Ed. Growth and Maturation Factors (John Wiley, New York, 1984). Accordingly these references are incorporated by reference for their descriptions of TCGF activity assays. Generally, the TCGF assays are based on the ability of a factor to promote the proliferation of peripheral T lymphocytes or IL-2 dependent T cell lines, e.g. Gillis et al. J. Immunol., Vol. 120, pg. 2027 (1978). Proliferation can be determined by standard techniques, e.g. tritiated thymidine incorporation, or by colorimetric methods, Mosmann, J. Immunol. Meth., Vol. 65, pgs. 55-63 (1983).

By way of example, human TCGF activity can be assayed by the following steps: (1) washed human peripheral blood lymphocytes (about $2 \times 10^5$ in 50 microliters) previously stimulated with phytohemagglutinin (PHA) for 7 days and subsequently cultured for 7 days with IL-2 are added to a microtiter well; (2) dilutions (50 microliter) of the TCGF-containing material are added to each well; (3) the lymphocytes are incubated 72 hours at 37°C; (4) tritiated thymidine (about 20 microliters, 20 microcuries/ml) is added to each well; and (5) cells are harvested onto filter paper strips, washed, and counted in a scintillation counter.

Units of human IL-4 TCGF activity are defined with respect to supernatants of COS 7 monkey cells (ATCC CRL 1651) transfected by the plasmid pcD-human-IL4 (clone 125) (deposited with the ATCC under accession number 67029). Such supernatants are produced as follows. About $1 \times 10^6$ COS 7 cells are seeded onto 100 mm tissue culture plates containig Dulbecco's Modified Eagle's medium (DME), 10% fetal calf serum, and 4 mM L-glutamine. About 24 hours after seeding, the medium is aspirated from the plates and the cells are washed twice with serum free buffered (50 mM Tris) DME. To each plate is added 4 ml serum free buffered DME (with 4 mM L-glutamine), 80 microliters DEAE-dextran, and 5 micrograms of pcD-125 DNA. The cells are incubated in this mixture for 4 hours at 30°C, after which the mixture is aspirated off and the cells are washed once with serum free buffered DME. After washing, 5 ml of DME with 4 mM L-glutamine, 100 micromolar chloroquine, and 2% fetal calf serum is added to each plate, the cells are incubated for 3 hours, and then twice washed with serum free buffered DME. Next, 5 ml DME with 4 mM L-glutamine and 4% fetal calf serum is added and the cells are incubated at 37°C for 24 hours. Afterwards the cells are washed 1-3 times with DME or PBS, 5 ml serum free DME (with 4 mM L-glutamine) is added, and the cells are incubated at 37°C until culture supernatants are harvested 5 days later.

One unit, as used herein, is the amount of factor which is one well (0.1 ml) stimulates 50% maximal proliferation of $2 \times 10^4$ PHA stimulated PBLs over a 48 hour period.

## EXAMPLES

The following examples serve to illustrate the present invention. Selection of vectors and hosts as well as the concentration of reagents, temperatures, and the values of other variable parameters are only to exemplify application of the present invention and are not to be considered as limitations thereof.

## EXAMPLE 1

### Expression of IL-4 in Escherichia coli

An E. coli expression vector, denoted TRPC11, was constructed using standard techniques, e.g. as disclosed in Maniatis et al., Molecular Cloning: A Laboratory Manual (Cold Spring Harbor Laboratory, New York, 1982).

The TRPC11 vector was constructed by ligating a synthetic consensus RBS fragment to ClaI linkers (ATGCAT) and by cloning the resulting fragments into ClaI restricted pMT11hc (which had been previously modified to contain the ClaI site). pMT11hc is a small (2.3 kilobase) high copy, AMP$^R$, TET$^S$ derivative of pBR322 that bears the πVX plasmid (described by Maniatis et al., cited above) EcoRI-HindIII polylinker region. It was modified to contain the ClaI site by restricting pMTIIhc with EcoRI and BamHI, filling in the resulting sticky ends and ligating with ClaI linker (CATCGATG), thereby restoring the EcoRI and BamHI sites and replacing the SmaI site with a ClaI site.

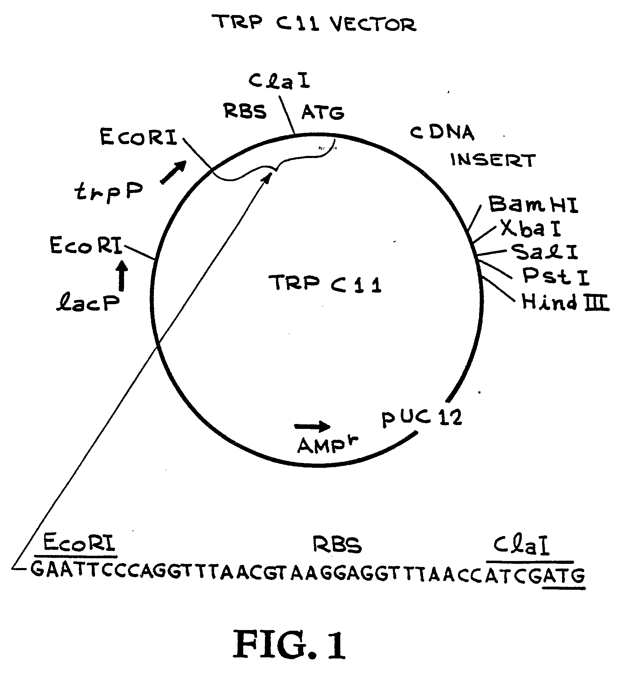

One transformant from the TRPC11 construction had a tandem RBS sequence flanked by ClaI sites. One of the ClaI sites and part of the second copy of the RBS sequence were removed by digesting this plasmid with PstI, treating with Bal31 nuclease, restricting with EcoRI, and treating with T4 DNA polymerase in the presence of all four deoxynucleotide triphosphates. The resulting 30-40 bp fragments were recovered via polyanylamide gelelectrophoresis and cloned into SmaI restricted pUC12. A 248 bp E. coli trpP-bearing EcoRI fragment derived from pKC101 (described by Nichols et al. in Methods in Enzymology, Vol. 101, pg. 155 (Academic Press, N.Y. 1983)) was then cloned into the EcoRI site to complete the TRPC11 construction, which is illustrated in Figure 1.

TRPC11 was employed as a vector for human IL-4 cDNA by first digesting it with ClaI and BamHI, purifying it, and then mixing it with the EcoRV/BamHI fragment of pcD-125 (deposited with the ATCC under accession number 67029) in a standard ligation solution containing 0.1 micromolar of the following synthetic linker:

5' - CG ATG CAC AAG TGC GAT - 3'
    TAC GTG TTC ACG CTA

E. coli AB1899 were transformed directly with the ligation solution using the standard calcium chloride procedure, propagated, and plated. Colonies containing the IL-4 cDNA insert were selected using a labeled oligonucleotide probe. The transformants were cultured in L-broth, and IL-4 was expressed constitutively.

## EXAMPLE 2

### Purification of Biologically Active IL-4 from Aggregates Produced by E. coli

A one liter culture of E. coli AB1899 (lon$^-$) (obtained from Yale University E. coli Genetics Center, New Haven CT), was grown to OD$_{560}$ = 2 (about $1.6 \times 10^9$ cells/ml). Cells were harvested by centrifugation at 4500 Xg for 15 minutes at 4°C. The pellets were resuspended in 30 ml of 50 mM Tris buffer, pH 8.0, containing 50 mM NaCl, lmM ethylenediaminetetracetic acid (EDTA), and 0.1 mM phenylmethylsulfonyl fluoride (PMSF). EDTA and PMSF are added to inhibit protease activity which might degrade the human IL-4 before purification. Next, the cells were sonicated, 50 pulses (50%) at 70 watts, and centrifuged, 25,000 Xg for 15 minutes at 4°C. The major protein component of the resulting pellet was shown to be IL-4 by comparing the gel band pattern of electrophoretically separated pellet material (which had been solubilized in sodium dodecylsulphate (SDS) and stained with Coomassie Blue) with a negative control.

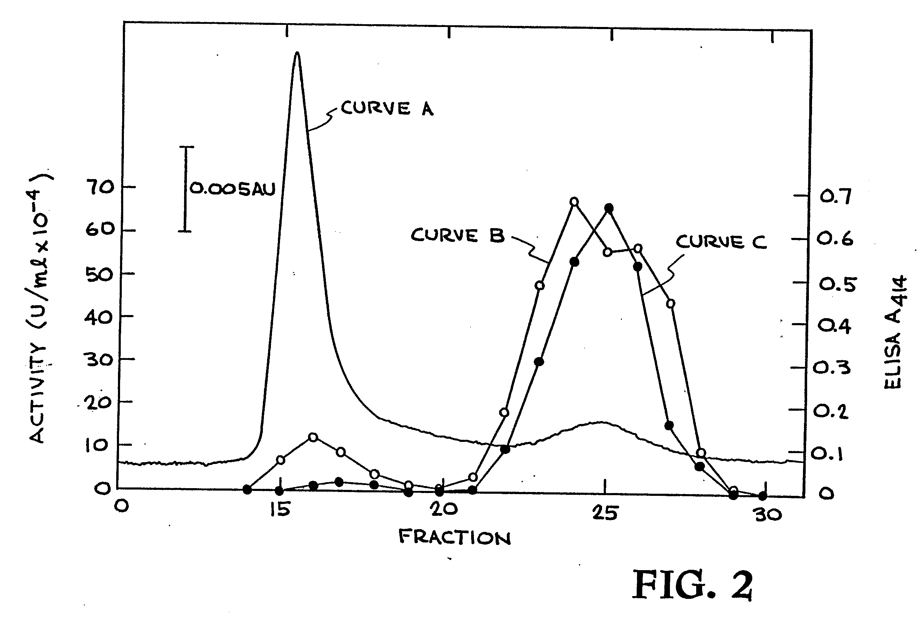

After removal of the supernatant, pellet material was resuspended (9 ml for each gram of pellet material in Tris buffer solution (50 mM Tris, 50 mM NaCl, 1 mM EDTA, 0.1 mM PMSF, pH 8.0) containing 5M guanidine HCl, 2 mM glutathione (reduced form), and 0.2 mM glutathione (oxidized form). After approximately 1 hour at room temperature, the solution was diluted 1:9 into a Tris buffer solution, pH 8.0, containing 2 mM glutathione (reduced form) and 0.2 mM glutathione (oxidized form). Whenever precipitates occurred during the dilution, dialysis, or concentration steps, they were removed by centrifugation before proceeding. The entire volume was then dialyzed overnight 3 times against 3 liters of phosphate buffer solution. The dialyzate (i.e. the material retained by the dialysis bag) was concentrated by an Amicon YM5 filter (final concentration 8 mg/ml), and subjected to gel filtration chromatography (column: P30 (BioRad), 1.5 x 90 cm; PBS elution buffer; flow rate: 8 ml/hr). Fractions were collected over 15 minute intervals. Fractions 23-27 (see Figure 2) were pooled and further analyzed by reverse phase HPLC. Such analysis indicated that the pooled fractions contained greater than 95% pure human IL-4. The yield from the 1 liter culture ($OD_{560}$ of 2) was 2 mg of human IL-4 with a specific activity of $5 \times 10^7$ units/mg.

Figure 2 illustrates the elution profiles for total protein (curve A), IL-4 (curve B), and biological activity (curve C). Protein content was measured by optical density at 280 nm. IL-4 content was measured by ELISA e.g. Tijssen, Practice and Theory of Enzyme Immunoassays (Elsevier, New York, 1985), using a monoclonal antibody against human IL-4 (expressed by mammalian cells). Biological activity was monitored by measuring T cell growth factor (TCGF) activity of the successive fractions eluted from the gel filtration column.

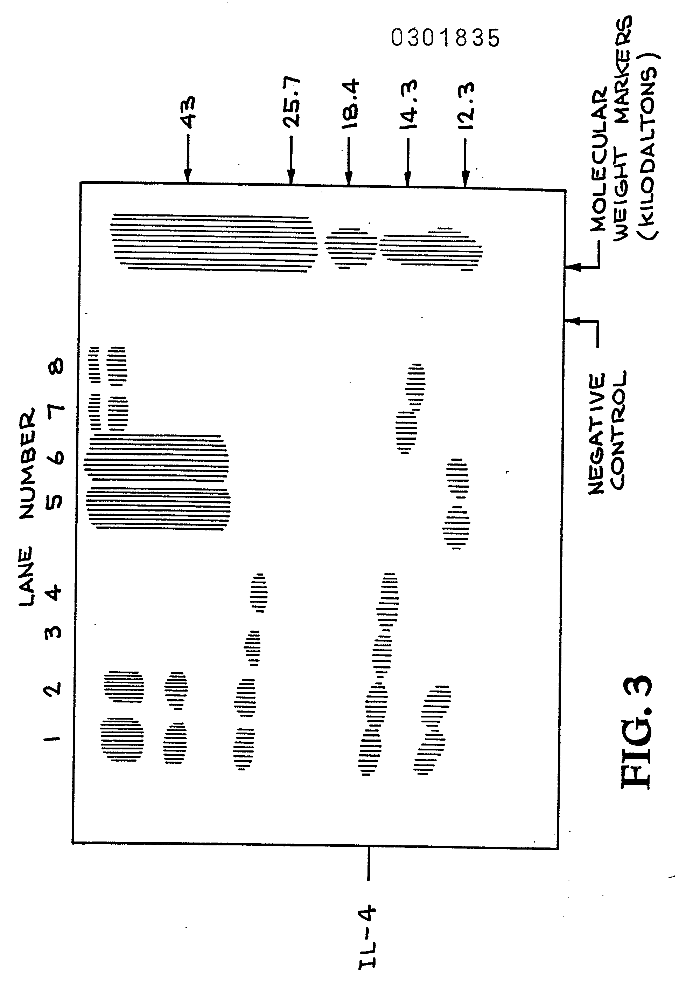

The results obtained by the steps of the above procedure prior to gel filtration were compared to those obtained in alternative procedures in which the chaotropic agent was 8M urea and in which the reducing agent was present or absent during the solubilization step of the procedure. Comparisons were made on the basis of gel positions of IL-4 electrophoretically separated and detected by western blotting, e.g. Burnette, Anal. Biochem., Vol. 112, pgs. 195-203 (1981), using rat antisera against a conjugate between an N-terminal 12-mer peptide of human IL-4 and myoglobin. The gel positions are illustrated in Figure 3. Proteins of lanes 1-4 were separated under reducing conditions (betamercaptoethanol), and proteins of lanes 5-9 were separated under non-reducing conditions (no betamercaptoethanol). The proteins of lanes 1, 2, 5, and 6 were solubilized using 8M urea as the chaotropic agent, and the proteins of lanes 3, 4, 7, and 8 were solubilized using 5M guanidine HCl as the chaotropic agent. In odd numbered lanes, proteins were solubilized without the presence of a reducing agent, and in even numbered lanes, proteins were solubilized in the presence of the reducing agent, glutathione. Lane 9 is a negative control consisting of Trp CII-mIL-2 expressed and refolded protein. Comparison of the lanes where urea was used as the chaotropic agent to those where guanidine was used demonstrates that significantly better results are obtained with guanidine, either with or without a reducing agent.

The descriptions of the foregoing embodiments of the invention have been presented for purpose of illustration and description. They are not intended to be exhaustive or to limit the invention to the precise forms disclosed, and obviously many modifications and variations are possible in light of the above teaching. The embodiments were chosen and described in order to best explain the principles of the invention and its practical application to thereby enable others skilled in the art to best utilize explain the principles of the invention and its practical application to thereby enable others skilled in the art to best utilize the invention in various embodiments and with various modifications as are suited to the particular use contemplated. It is intended that the scope of the invention be defined by the claims appended hereto.

**Claims**

1. A method for recovering biologically active human interleukin-4 molecule from insoluble aggregates in bacteria, the method comprising the steps of:
disrupting the cell membrane of the bacteria;
separating the insoluble aggregates from the disruptate;
solubilizing the insoluble aggregates with a first aqueous solution containing a chaotropic agent and a reducing agent to form reduced and denatured human interleukin-4;
diluting the concentration of the reduced and denatured human interleukin-4 in the first aqueous solution with a second aqueous solution containing a reducing agent to reduce the interactions between different parts of the same interleukin-4 molecule;
reducing the concentration of the chaotropic agent and the concentration of the reducing agent so that the reduced and denatured human interleukin-4 renatures and oxidizes to form biologically active human interleukin-4; and
purifying the biologically active human interleukin-4.

2. The method of claim 1 wherein said chaotropic agent is guanidine HCl.

3. The method of claim 1 wherein said reducing agent comprises a thiol reducing agent which is glutathione or dithiothreitol.

4. The method of claim 3 wherein said reducing agent consists of 1-3 mM of the reduced form of glutathione and 0.1-0.6 mM of the oxidized form of glutathione.

5. The method of claims 2, 3 or 4 wherein said step of solubilizing includes forming a solution of said insoluble aggregates in said first aqueous solution at a concentration of less than about 2.5 mg/ml.

6. The method of claims 2, 3 or 4 wherein said step of diluting with a second aqueous solution results in a 1.5-3.0 fold increase in the average intermolecular distance between reduced and denatured human interluekin-4 molecules.

7. The method of claim 6 wherein the second aqueous solution contains salts at a concentration of about 50 to about 200 mM or greater.

8. The method of claim 7 wherein the concentrations of the chaotropic agent and the reducing agent after dilution with the second aqueous solution are reduced up to a factor of about a thousand by dialysis.

9. The method of claim 7 wherein said step of purifying is accomplished by gel filtration chromatography.

10. The method of claim 1 wherein said step of purifying yields human interleukin-4 having a specific activity of at least $5 \times 10^7$ units/mg.

0301835

TRP C11 VECTOR

FIG. 1

FIG. 2

FIG. 3

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 156 373 (CETUS CORP.) * Claims; page 5, line 17 - page 7, line 29 * | 1-10 | C 12 P    1/02 |
| Y | WO-A-8 702 990 (SCHERING BIOTECH) * Claims; page 43, lines 19-30; page 79, lines 16-23 * | 1-10 | |
| Y | BIOCHEMISTRY, vol. 26, no. 11, 2nd June 1987, pages 3129-3134, American Chemical Society, Washington, DC, US; T. TSUJI et al.: "Characterization of disulfide bonds in recombinant proteins: reduced human interleukin 2 in inclusion bodies and its oxidative refolding" * Whole article * | 1-10 | |
| Y | WO-A-8 304 418 (CELLTECH LTD) * Claims * | 1-10 | |
| P,X | EUROPEAN JOURNAL OF BIOCHEMISTRY, vol. 173, no. 1, 5th April 1988, pages 109-114, FEBS, Berlin, DE; A. VAN KIMMENADE et al.: "Expression, renaturation and purification of recombinant human interleukin 4 from Escherichia coli" * Whole article * | 1-10 | TECHNICAL FIELDS SEARCHED (Int. Cl.4) C 12 P C 12 N |

The present search report has been drawn up for all claims

| Place of search | Date of completion of the search | Examiner |
|---|---|---|
| THE HAGUE | 27-10-1988 | HUBER-MACK A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P0401)